# EUROPEAN PATENT SPECIFICATION

(11) **EP 3 721 819 B1**
(45) Date of publication and mention of the grant of the patent: **22.01.2025**
(21) Application number: 17934253.0
(22) Date of filing: 07.12.2017
(51) Int. Cl.: A61B 17/32, A61B 18/04

(54) **ULTRASONIC VIBRATION PROPAGATION ASSEMBLY**
ULTRASCHALLSCHWINGUNGSAUSBREITUNGSANORDNUNG
ENSEMBLE DE PROPAGATION DE VIBRATIONS ULTRASONORES

(43) Date of publication of application: 14.10.2020
(73) Proprietor: Beijing SMTP Technology Co., Ltd., Beijing 100083 (CN)
(72) Inventor: CAO, Qun, Beijing 100083 (CN); HU, Xiaoming, Beijing 100083 (CN); ZHAN, Songtao, Beijing 100083 (CN); FENG, Zhen, Beijing 100083 (CN); LI, Chunyuan, Beijing 100083 (CN)
(74) Representative: Beyer, Andreas
(86) International application number: PCT/CN2017/115037
(87) International publication number: WO 2019/109308

(56) References cited:
- WO-A1-2017/027853
- WO-A1-2017/027853
- CN-A- 103 596 510
- CN-A- 104 540 461
- CN-A- 105 310 746
- CN-A- 107 466 226
- US-A1- 2001 047 166
- US-A1- 2003 125 620
- US-A1- 2007 060 926
- US-A1- 2011 278 988

## Description

### Technical Field

The present disclosure relates to the technical field of medical instruments, and in particular to an ultrasonic scalpel bit, an ultrasonic vibration propagation assembly, and an ultrasonic hemostasis and cutting system.

### Background

Compared with ordinary scalpels and other energy surgical instruments, ultrasonic hemostasis and cutting systems have advantages such as easy operation, small surgical trauma area, no smoke, less amount of bleeding, high precision of surgery, and quick postoperative recovery, and have been widely used in surgery.

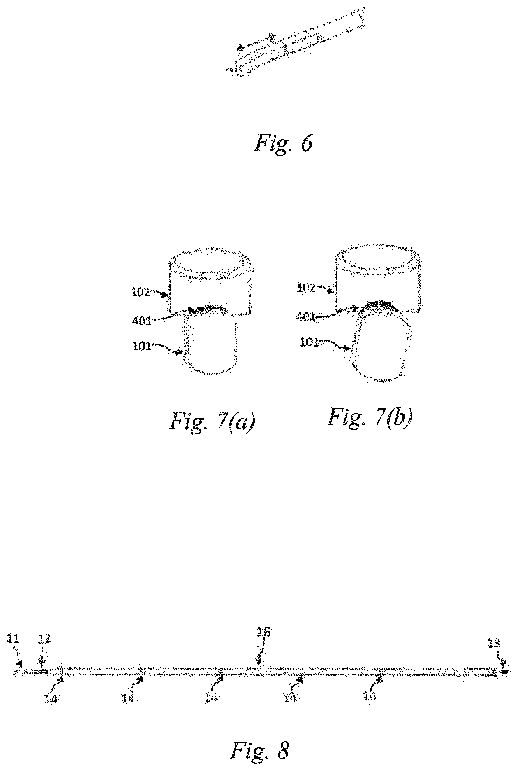

During coagulation or cutting, microscopic temperature uniformity of the ultrasonic hemostasis and cutting systems mainly depends on the temperature diffusion rate inside tissues and the uniformity of frictional heat generation caused by ultrasonic vibrations. Ultrasonic scalpel tips of existing ultrasonic hemostasis and cutting systems are straight cutters, and the ultrasonic scalpel bits only produce longitudinal vibrations. When the ultrasonic scalpel tip is in a balanced position, a gap between the ultrasonic scalpel tip and a clamping arm is at the minimum, and the clamping pressure of the ultrasonic scalpel tip and the clamping arm on a biological tissue is maximized, as shown in Fig. 7(a). According to the principle of longitudinal ultrasonic vibration, the further away from the ultrasonic scalpel tip, the smaller the longitudinal amplitude. When the ultrasonic scalpel is used to cut the biological tissue, the clamping pressure of the scalpel tip and the end of the scalpel on the tissue will also vary with the thickness of the clamped tissue, the change in the position where the tissue is clamped by the ultrasonic scalpel bit, and the change in the elasticity of the tissue after heating and denaturation. As a result, the heat generated by the ultrasonic vibration differs a lot, and the temperature inside the tissue being cut or coagulated is not uniform, so that there is a relatively large difference in the coagulation and cutting effects of the ultrasonic scalpel bit at different positions. For example, when hemostasis or cutting is performed on a biological tissue such as a large-diameter blood vessel, there is a condition in which the hemostasis or cutting has been done at the position being cut by the scalpel tip, but the hemostasis has not yet done at the remaining positions. In this case, if the cutting operation is still continued, the tissue at the position being cut by the scalpel tip may be overheated to cause adverse effects such as high-temperature carbonization, which may endanger the safety of a patient.

US 2007/0060926 A1 discloses an ophthalmologic cutting device having a base support section and a tip with a blade section. The blade section has upper and lower edges, and a forward aspiration free edge extending between them, with the upper edge having a shorter longitudinal length compared with the lower edge and where the forward edge slopes down from a distal end of the upper edge to a distal end of the lower edge.

US 2011/0278988 A1 discloses an apparatus for generating torsional-mode ultrasonic vibrations to activate an ultrasonically-vibratable tool. A horn is mounted coaxially to a distal face of a first transducer unit. A thin, elongate, cylindrical waveguide extends from the horn to an end effector at a distal tip of the tool. There are a series of nodes along the waveguide.

WO 2017/027853 A1 discloses an ultrasonic surgical device including an elongate waveguide having a longitudinal axis and a distal end, and a blade extending away from the distal end of the waveguide, the blade including a curved portion that has at least five faces extending lengthwise along at least a portion of the length of the blade.

US 2003/0125620 A1 discloses a surgical instrument comprising an ultrasonic oscillation mechanism for generating an ultrasonic vibration, a horn coupled with the ultrasonic oscillation mechanism for amplifying the vibration transmitted from the ultrasonic oscillation mechanism, a vibration conversion mechanism for converting the vibration transmitted from the ultrasonic oscillation mechanism into a composite vibration.

US 2001/0047166 A1 discloses an ultrasonic tissue dissection system providing combined longitudinal and torsional motion of tips, together with irrigation and aspiration, for improved cutting of resistant biological tissue.

### Summary of the Disclosure

The present disclosure provides an ultrasonic vibration propagation assembly, and an ultrasonic hemostasis and cutting system, in order to solve the problem of poor cutting and hemostasis effects on biological tissues such as large blood vessels in the prior art.

The objective of the present invention may be achieved by the subject matters according to the independent claim.

Advantageous embodiments of the present invention are described by the dependent claims.

In a first aspect, the present disclosure provides an ultrasonic vibration propagation assembly according to claim 1.

Further, the vibration guide groove may be configured as a beveled groove, which advances spirally along an ultrasound axis, and pitch of which is uniform or non-uniform.

Further, the ratio of the pitch of the vibration guide groove to the wavelength of an ultrasonic vibration may be 0.2 ~ 2.

Further, the beveled groove may be in the shape of a trapezoid, a semi-circle or a triangle.

Further, a plurality of beveled grooves may be provided.

Further, the beveled grooves may be arranged on the ultrasonic scalpel bit at equal intervals.

Further, the ultrasonic scalpel tip may be a gradually-varying width in addition to being laterally bent at the pointed end thereof.

Further, the gradually-varying width may be a trapezoidal gradually-varying width. Further, the support structure of the ultrasonic vibration propagation assembly may further comprise an outer sleeve, an inner sleeve and a lubrication cylinder, wherein the ultrasonic scalpel bit is located inside the lubrication cylinder.

Further, the lubrication cylinder may be a polytetrafluoroethylene casing.

In a second aspect, the present disclosure further provides an ultrasonic hemostasis and cutting system according to claim 11.

Further, the ultrasonic hemostasis and cutting system may further comprise a host, a handle, an ultrasonic transducer and a foot switch or a button, wherein the handle comprises a clamping switch, the ultrasonic scalpel bit of the ultrasonic vibration propagation assembly is removably connected to the ultrasonic transducer via a connection portion at a rear end of the ultrasonic scalpel bit, and the host is electrically connected to the ultrasonic transducer via a cable.

In the present disclosure, by designing the bit of the ultrasonic hemostasis and cutting system in a bent shape and converting a longitudinal ultrasonic vibration into a longitudinal-torsional composite vibration, on the one hand, the dependence of the temperature uniformity inside the tissue on the vibration direction is reduced, and the effective length of the vibration friction is increased; and on the other hand, when the ultrasonic scalpel bit in the bent shape is subjected to a torsional vibration, the clamping pressures of the clamping arm are different at positions with different distances from the scalpel tip. The clamping pressure is greatly reduced in an area near the scalpel tip, whereas the pressure is reduced less in an area remote from the scalpel tip. Therefore, the temperature uniformity inside the tissue being cut or coagulated is improved, thereby improving the efficiency and safety of cutting and hemostasis.

In the present disclosure, the frictional heat generation effect between the vibration guide groove and the lubrication cylinder is further reduced by providing the vibration guide groove at the rear end of the ultrasonic scalpel tip. In addition, the assembly process is simplified by designing a polytetrafluoroethylene casing in the support structure of the ultrasonic vibration propagation assembly, thereby reducing assembly time.

In the present disclosure, the mass distribution law of the ultrasonic scalpel tip along a vibration axis is changed by making the ultrasonic scalpel bit have a gradually-varying width, such that the amplitude and pressure distribution characteristics of the ultrasonic scalpel tip along the vibration axis are improved, further improving the temperature uniformity of the ultrasonic scalpel tip during coagulation or cutting of the biological tissue, thereby improving the hemostasis effect.

### Brief Description of the Drawings

Figs. 1(a)-1(d) are schematic structural views of an ultrasonic scalpel bit of an ultrasonic vibration propagation assembly of Embodiment 1 of the present disclosure;
Figs. 2(a)-2(d) are schematic views of the shape of an ultrasonic vibration guide groove of an ultrasonic vibration propagation assembly of Embodiment 1 of the present disclosure;
Fig. 3 is a schematic structural view of an ultrasonic vibration propagation assembly of Embodiment 1 of the present disclosure;
Fig. 4 is a schematic view of an ultrasonic hemostasis and cutting system of Embodiment 1 of the present disclosure;
Fig. 5 is a schematic view of a biological tissue clamped by the ultrasonic hemostasis and cutting system of Embodiment 1 of the present disclosure;
Fig. 6 is a schematic view of a composite vibration formed from longitudinal and torsional vibrations of the ultrasonic scalpel bit of an ultrasonic vibration propagation assembly of Embodiment 1 of the present disclosure;
Fig. 7 (a) is a schematic view of a gap between a clamping arm and an existing ultrasonic scalpel tip;
Fig. 7 (b) is a schematic view of a gap between a clamping arm and an ultrasonic scalpel tip of Embodiment 1 of the present disclosure;
Fig. 8 is a schematic structural view of an ultrasonic scalpel bit of Embodiment 2 of the present disclosure;
Fig. 9(a) is a top view of an ultrasonic scalpel tip having a trapezoidal gradually-varying width of Embodiment 3 of the present disclosure; and
Fig. 9(b) is a side view of the ultrasonic scalpel tip having the trapezoidal gradually-varying width of Embodiment 3 of the present disclosure.

### Detailed Description of Embodiments

In the following embodiments, the detailed description and the drawings illustrate in conjunction how the disclosed embodiments are implemented. It is to be understood that other embodiments are feasible, and the embodiments may be modified structurally or logically without departing from the scope of the appended claims. The scope of the invention is defined by the appended claims.

### Embodiment 1:

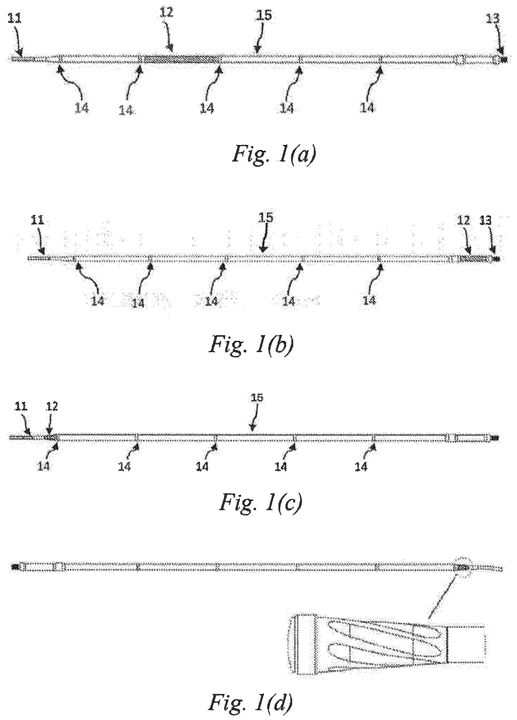

An ultrasonic scalpel bit 101 is shown, the structure of which is as shown in Figs. 1(a), 1(b), 1(c), 6 and 8, comprising an ultrasonic scalpel tip 11, a waveguide 15, a connection portion 13 and vibration node bosses 14, wherein the ultrasonic scalpel tip 11 is arranged in front of the waveguide 15, the connection portion 13 is arranged behind the waveguide 15, the vibration node bosses 14 are arranged on the waveguide 15, the ultrasonic scalpel tip 11 is laterally bent at a pointed end thereof, and a vibration guide groove 12 is provided on the waveguide 15. Among the above components, the ultrasonic scalpel tip 11 is used to perform cutting and hemostasis operations on a biological tissue 401; the vibration guide groove 12 is used to convert a longitudinal vibration of an ultrasonic transducer into a longitudinal-torsional composite vibration; the connection portion 13 is used to connect the ultrasonic scalpel bit to the ultrasonic transducer to achieve continuous propagation of ultrasonic vibration, which is convenient for a surgeon to perform cleaning and disinfection operations, and the connection portion 13 may be connection threads; the vibration node bosses 14 are used to support the ultrasonic scalpel bit 101 and reduce the friction between the ultrasonic scalpel bit 101 and the support structure during vibration, and the number of vibration node bosses is not limited; and the waveguide 15 is used to propagate the ultrasonic vibration. The vibration guide groove 12 may be provided between two vibration node bosses 14 of the waveguide 15, or may be provided at another location on the waveguide 15.

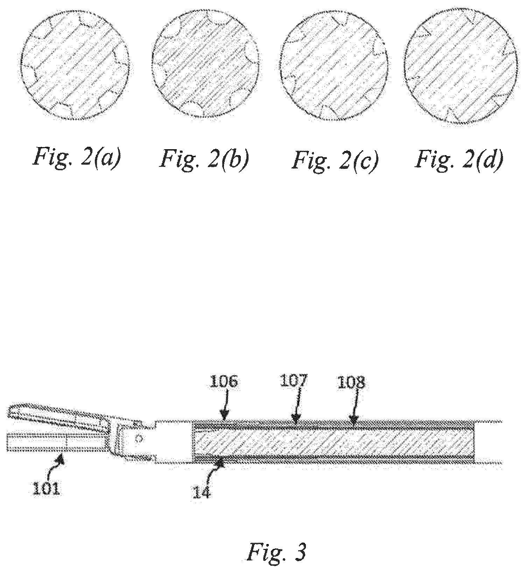

Further, as shown in Fig. 1(d), the ultrasonic vibration guide groove 12 is configured as beveled groove which advances spirally along an ultrasound axis, and the shape of the beveled groove may be a trapezoid, a semi-circle or a triangle, as shown in Figs. 2(a)-(d). Pitch may be uniform or non-uniform. Preferably, the ratio of the pitch of the guide groove to the wavelength of the ultrasonic vibration is 0.2 ~ 2. The number of the guide grooves is at least one. Preferably, the guide grooves are arranged at equal intervals in a circumferential direction of the ultrasonic scalpel bit.

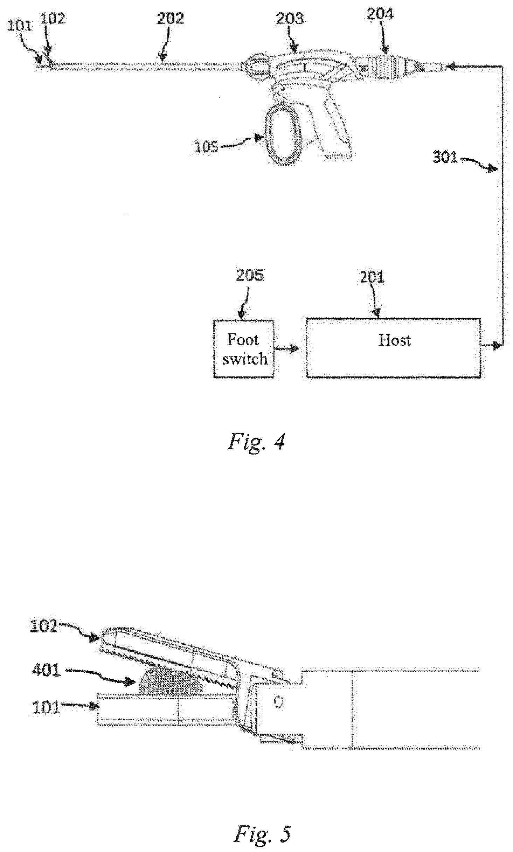

Further, as shown in Figs. 3 and 5, an ultrasonic vibration propagation assembly is further disclosed in this embodiment, which comprises an ultrasonic scalpel bit 101, a support structure and a clamping arm 102. The clamping arm 102 is located at a front end of the support structure. The support structure comprises an outer sleeve 106, an inner sleeve 107, and a lubrication cylinder 108. A rear end of the ultrasonic scalpel bit 101 is located inside the lubrication cylinder 108. The inner sleeve 107 and the outer sleeve 106 are sheathed outside the lubrication cylinder 108. The vibration node bosses 14 can form a support for the ultrasonic scalpel bit together with the lubrication cylinder 108, the inner sleeve 107 and the outer sleeve 106, and can also reduce the heat generation caused by the friction between the ultrasonic scalpel bit and the support member during vibration. In addition, the lubrication cylinder 108 may also be made of a low-friction-coefficient material such as a polytetrafluoroethylene casing, to further reduce the friction. At the same time, the use of a polytetrafluoroethylene casing can simplify the assembly process and reduce the time taken for assembly compared to the prior art in which each vibration node boss 14 is provided with a collar.

In addition, as shown in Fig. 4, this embodiment further comprises an ultrasonic hemostasis and cutting system, comprising a host 201, an ultrasonic vibration propagation assembly 202, a handle 203, an ultrasonic transducer 204, and a foot switch or a button 205, wherein the handle 203 comprises a clamping switch 105, the host 201 is connected to the ultrasonic transducer 204 via a cable 301, and the ultrasonic scalpel bit 101 of the ultrasonic vibration propagation assembly 202 is removably connected to the ultrasonic transducer 204 via a connection portion 13 at a rear end of the ultrasonic scalpel bit. The ultrasonic transducer 204 is used to convert a high-voltage electrical signal into an ultrasound vibration to drive the ultrasonic scalpel bit to operate. The host 201 is used to detect the access of the ultrasonic handle, control and adjust an ultrasonic driving signal so that the ultrasonic system can operate at the optimal resonance frequency, and also identify and detect the vibration state of the handle, such as identifying current, voltage and phase parameters of the ultrasonic driving signal, and detecting whether the driving signal is over-current, open-circuit or short-circuit. The host 201 can enable a user to perform the starting, stopping and other operations by means of the foot switch or the button 205, to control the output and stop of ultrasound, and can also achieve the functions such as the setting of the output power of the ultrasonic hemostatic scalpel, fault diagnosis and warning by means of a user's operation interface. The surgeon manipulates the clamping switch 105, driving the clamping arm 102 of the ultrasonic vibration propagation assembly 202 to rotate via the outer sleeve 106 and the inner sleeve 107. The clamping arm 102 and the ultrasonic scalpel tip 11 together perform the clamping operation on the biological tissue 401, as shown in Fig. 5.

In this embodiment, by providing the vibration guide groove 12 on the ultrasonic scalpel bit 101, the longitudinal vibration of the ultrasonic transducer can be converted into the longitudinal-torsional composite vibration, so that the ultrasonic scalpel bit is longitudinally vibrated and twisted at the same time, to form a composite vibration, as shown in Fig. 6. At the same time, since the ultrasonic scalpel tip 11 is laterally bent at the pointed end portion thereof, the size of the gap between the ultrasonic scalpel tip 11 and the clamping arm 102 varies periodically with the change of the vibration and torsion when the bent ultrasonic scalpel tip in this embodiment is used to cooperate with the longitudinal-torsional composite vibration. When the torsional vibration reaches the maximum displacement, the gap between the ultrasonic scalpel tip 11 and the clamping arm 102 is the largest, as shown in Fig. 7(b), at which time the clamping pressure of the ultrasonic scalpel tip 11 and the clamping arm 102 on the biological tissue is minimized, and the difference in the clamping strength between the scalpel tip portion and the other portions of the bit is reduced compared to the prior art ultrasonic scalpel which uses a straight bit and does not generate a torsional vibration. As a result, the difference in the amount of the generated heat is reduced, and the temperature inside the tissue being cut or coagulated is more uniform, which is conducive to closing the biological tissue such as a large blood vessel.

### Embodiment 2:

As shown in Fig. 8, different from Embodiment 1 in which the vibration guide groove is provided on the waveguide 15, in this embodiment, the ultrasound guide groove 12 of the ultrasonic scalpel bit 101 is provided on the ultrasonic scalpel tip 11, and is located at a rear end of the ultrasonic scalpel tip 11. The frictional heat generation effect between the vibration guide groove 12 and the lubrication cylinder 108 can be further reduced by means of the above arrangement.

### Embodiment 3:

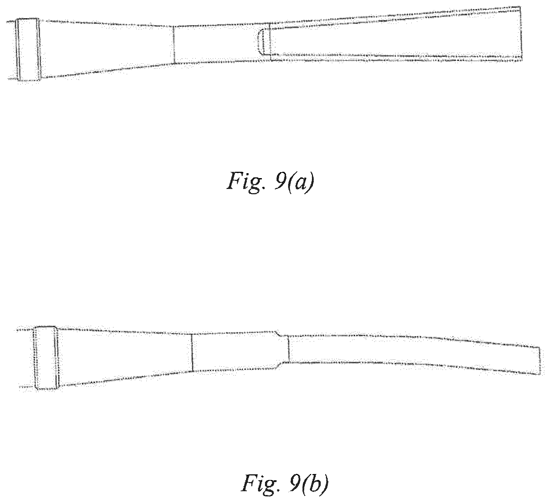

This embodiment further defines the shape of the ultrasonic scalpel tip 11 of the ultrasonic scalpel bit 101 on the basis of Embodiment 1 or 2. That is, the ultrasonic scalpel tip 11 comprises a gradually-varying width in addition to being laterally bent at the pointed end thereof. For example, the gradually-varying width may be a trapezoidal gradually-varying width, a top view of which is as shown in Fig. 9(a) in which the front end of the ultrasonic scalpel tip 11 has a width larger than that of the rear end thereof, and a side view of which is as shown in Fig. 9(b). With the above design, the mass distribution law of the ultrasonic scalpel tip 11 along a vibration axis can be changed, such that the amplitude and pressure distribution characteristics of the ultrasonic scalpel tip 11 along the vibration axis are improved, further improving the temperature uniformity of the ultrasonic scalpel tip 11 during coagulation or cutting of the biological tissue 401, thereby improving the hemostasis effect.

Although various embodiments have been described in detail above, those skilled in the art will appreciate that various alternative embodiments may be used to substitute for the specific disclosure of the embodiments mentioned above if they are in accordance with the appended claims.

## Claims

1. An ultrasonic vibration propagation assembly (202), comprising:
a support structure;
a clamping arm (102) located at a front end of the support structure; and
an ultrasonic scalpel bit (101), a portion of the ultrasonic scalpel bit (101) being located in the support structure, the ultrasonic scalpel bit (101) comprising an ultrasonic scalpel tip (11) for performing operations on biological tissue and configured to perform a clamping operation together with the clamping arm (102), a connection portion (13), and a waveguide (15), wherein the ultrasonic scalpel tip (11) is arranged in front of the waveguide (15), and the connection portion (13) is arranged behind the waveguide (15),
**characterized in that** the ultrasonic scalpel bit further comprises vibration node bosses (14), wherein the vibration node bosses (14) are arranged on the waveguide (15), wherein the ultrasonic scalpel tip (11) is laterally bent at a pointed end thereof with respect to the clamping arm, and wherein a vibration guide groove (12) is further provided on the ultrasonic scalpel bit (101), and
wherein the vibration guide groove (12) is located on the ultrasonic scalpel tip (11), at a rear end of the ultrasonic scalpel tip (11), or the vibration guide groove is provided on the waveguide (15) and is located between two vibration node bosses (14).

2. The ultrasonic vibration propagation assembly (202) of claim 1, wherein the vibration guide groove (12) is configured as a beveled groove, which advances spirally along an ultrasound axis, and a pitch of which is uniform or non-uniform.

3. The ultrasonic vibration propagation assembly (202) of claim 2, wherein the ratio of the pitch of the vibration guide groove (12) to the wavelength of an ultrasonic vibration is 0.2 ~ 2.

4. The ultrasonic vibration propagation assembly (202) of claim 2 or 3, wherein the beveled groove is in the shape of a trapezoid, a semi-circle or a triangle.

5. The ultrasonic vibration propagation assembly (202) of any one of claims 2 to 4, comprising a plurality of beveled grooves.

6. The ultrasonic vibration propagation assembly (202) of claim 5, wherein the plurality of beveled grooves are arranged on the ultrasonic scalpel bit at equal intervals.

7. The ultrasonic vibration propagation assembly (202) of any one of claims 1 to 6, wherein the ultrasonic scalpel tip (11) comprises a gradually-varying width in addition to being laterally bent at the pointed end thereof.

8. The ultrasonic vibration propagation assembly (202) of claim 7, wherein the gradually-varying width is a trapezoidal gradually-varying width.

9. The ultrasonic vibration propagation assembly (202) of claim 1, wherein the support structure comprises an outer sleeve (106), an inner sleeve (107) and a lubrication cylinder (108), the ultrasonic scalpel bit (101) being located inside the lubrication cylinder (108).

10. The ultrasonic vibration propagation assembly of claim 9, wherein the lubrication cylinder (108) is a polytetrafluoroethylene casing.

11. An ultrasonic hemostasis and cutting system, comprising an ultrasonic vibration propagation assembly of any one of claims 1-10.

12. The ultrasonic hemostasis and cutting system of claim 11, further comprising a host (201), a handle (203), an ultrasonic transducer (204) and a foot switch or a button, wherein the handle (203) comprises a clamping switch (105), the ultrasonic scalpel bit (101) of the ultrasonic vibration propagation assembly is removably connected to the ultrasonic transducer (204) via a connection portion (13) at a rear end of the ultrasonic scalpel bit, and the host (201) is electrically connected to the ultrasonic transducer (204) via a cable.

## Patentansprüche

1. Ultraschallschwingungsausbreitungsbaugruppe (202), umfassend:
eine Trägerstruktur;
einem Klemmarm (102), der sich an einem vorderen Ende der Trägerstruktur befindet; und
einen Ultraschallskalpellaufsatz (101), wobei ein Teil des Ultraschallskalpellaufsatzes (101) in der Trägerstruktur befindlich ist, wobei der Ultraschallskalpellaufsatz (101) eine Ultraschallskalpellspitze (11) zur Durchführung von Operationen an biologischem Gewebe umfasst und so konfiguriert ist, dass er zusammen mit dem Klemmarm (102) eine Klemmoperation durchführt, einen Verbindungsabschnitt (13) und einen Wellenleiter (15), wobei die Ultraschallskalpellspitze (11) vor dem Wellenleiter (15) angeordnet ist und der Verbindungsabschnitt (13) hinter dem Wellenleiter (15) angeordnet ist,
**dadurch gekennzeichnet, dass** der Ultraschallskalpellaufsatz ferner Schwingungsknotenvorsprünge (14) umfasst, wobei die Schwingungsknotenvorsprünge (14) auf dem Wellenleiter (15) angeordnet sind, wobei die Ultraschallskalpellspitze (11) an ihrem spitzen Ende in Bezug auf den Klemmarm seitlich gebogen ist, und wobei eine Schwingungsführungsnut (12) ferner an dem Ultraschallskalpellaufsatz (101) vorgesehen ist, und
wobei sich die Schwingungsführungsnut (12) an der Ultraschallskalpellspitze (11) an einem hinteren Ende der Ultraschallskalpellspitze (11) befindet, oder die Schwingungsführungsnut an dem Wellenleiter (15) vorgesehen ist und zwischen zwei Schwingungsknotenvorsprüngen (14) befindlich ist.

2. Ultraschallschwingungsausbreitungsbaugruppe (202) nach Anspruch 1, wobei die Schwingungsführungsnut (12) als gefaste Nut ausgebildet ist, die spiralförmig entlang einer Ultraschallachse fortschreitet und deren Neigung gleichmäßig oder ungleichmäßig ist.

3. Ultraschallschwingungsausbreitungsbaugruppe (202) nach Anspruch 2, wobei das Verhältnis der Neigung der Schwingungsführungsnut (12) zur Wellenlänge einer Ultraschallschwingung 0,2 ~ 2 beträgt.

4. Ultraschallschwingungsausbreitungsbaugruppe (202) nach Anspruch 2 oder 3, wobei die gefaste Nut die Form eines Trapezoids, eines Halbkreises oder eines Dreiecks aufweist.

5. Ultraschallschwingungsausbreitungsbaugruppe (202) nach einem der Ansprüche 2 bis 4, umfassend mehrere gefaste Nuten.

6. Ultraschallschwingungsausbreitungsbaugruppe (202) nach Anspruch 5, wobei die mehreren gefaste Nuten in gleichen Abständen auf dem Ultraschallskalpellaufsatz angeordnet sind.

7. Ultraschallschwingungsausbreitungsbaugruppe (202) nach einem der Ansprüche 1 bis 6, wobei die Ultraschallskalpellspitze (11) zusätzlich zu ihrer seitlichen Biegung an ihrem spitzen Ende eine sukzessiv variierende Breite aufweist.

8. Ultraschallschwingungsausbreitungsbaugruppe (202) nach Anspruch 7, wobei die sukzessiv variierende Breite eine trapezoide sukzessiv variierende Breite ist.

9. Ultraschallschwingungsausbreitungsbaugruppe (202) nach Anspruch 1, wobei die Trägerstruktur eine äußere Hülle (106), eine innere Hülle (107) und einen Schmierzylinder (108) umfasst, wobei der Ultraschallskalpellaufsatz (101) innerhalb des Schmierzylinders (108) befindlich ist.

10. Ultraschallschwingungsausbreitungsbaugruppe nach Anspruch 9, wobei der Schmierzylinder (108) ein Gehäuse aus Polytetrafluorethylen ist.

11. Ultraschallhämostase- und schneidesystem, umfassend eine Ultraschallschwingungsausbreitungsbaugruppe nach einem der Ansprüche 1 bis 10.

12. Ultraschallhämostase- und schneidesystem nach Anspruch 11, ferner umfassend einen Host (201), einen Griff (203), einen Ultraschallwandler (204) und einen Fußschalter oder einen Knopf, wobei der Griff (203) einen Klemmschalter (105) umfasst, der Ultraschallskalpellaufsatz (101) der Ultraschallschwingungsausbreitungsbaugruppe über einen Verbindungsabschnitt (13) an einem hinteren Ende des Ultraschallskalpellaufsatzes abnehmbar mit dem Ultraschallwandler (204) verbunden ist, und der Host (201) über ein Kabel elektrisch mit dem Ultraschallwandler (204) verbunden ist.

## Revendications

1. Ensemble de propagation de vibrations ultrasonores (202) comprenant:
une structure de support;
un bras de serrage (102) situé à l'extrémité avant de la structure de support; et
un embout de scalpel ultrasonore (101), une partie de l'embout de scalpel ultrasonore (101) étant située dans la structure de support, l'embout de scalpel ultrasonore (101) comprenant une pointe de scalpel ultrasonore (11) pour effectuer des opérations sur des tissus biologiques et conçue pour effectuer une opération de serrage conjointement avec le bras de serrage (102), une partie de connexion (13) et un guide d'ondes (15), la pointe de scalpel ultrasonore (11) étant placée devant le guide d'ondes (15) et la partie de connexion (13) étant placée derrière le guide d'ondes (15),
**caractérisé en ce que** l'embout de scalpel ultrasonore comprend en outre des bossages de noeuds de vibration (14), les bossages de noeuds de vibration (14) étant disposés sur le guide d'ondes (15), dans lequel l'embout de scalpel ultrasonore (11) est plié latéralement à son extrémité pointue par rapport au bras de serrage, et dans lequel une rainure de guidage des vibrations (12) est en outre disposé sur l'embout de scalpel ultrasonore (101), et
dans lequel la rainure de guidage des vibrations (12) est située sur l'embout de scalpel ultrasonore (11), à une extrémité arrière de l'embout de scalpel ultrasonore (11), ou la rainure de guidage des vibrations est ménagée sur le guide d'ondes (15) et est située entre deux bossages de noeud de vibration (14)

2. Ensemble de propagation de vibrations ultrasonores (202) selon la revendication 1, dans lequel la rainure de guidage des vibrations (12) est conçue comme une rainure biseautée, qui avance en spirale le long d'un axe ultrasonore, et dont le pas est uniforme ou non uniforme.

3. Ensemble de propagation de vibrations ultrasonores (202) selon la revendication 2, dans lequel le rapport entre le pas de la rainure de guidage des vibrations (12) et la longueur d'onde d'une vibration ultrasonore est de 0,2 ~ 2.

4. Ensemble de propagation de vibrations ultrasonores (202) selon la revendication 2 ou 3, dans lequel la rainure biseautée se présente sous la forme d'un trapèze, d'un demi-cercle ou d'un triangle.

5. Ensemble de propagation de vibrations ultrasonores (202) selon l'une quelconque des revendications 2 à 4, comprenant une pluralité de rainures biseautées.

6. Ensemble de propagation de vibrations ultrasonores (202) selon la revendication 5, dans lequel la pluralité de rainures biseautées est disposée à intervalles égaux sur l'embout de scalpel ultrasonore.

7. Ensemble de propagation de vibrations ultrasonores (202) selon l'une quelconque des revendications 1 à 6, dans lequel l'embout de scalpel ultrasonore (11) comprend une largeur qui varie progressivement, en plus d'être courbée latéralement à son extrémité pointue.

8. Ensemble de propagation de vibrations ultrasonores (202) selon la revendication 7, dans lequel la largeur variable est une largeur variable trapézoïdale.

9. Ensemble de propagation de vibrations ultrasonores (202) selon la revendication 1, dans lequel la structure de support comprend un manchon extérieur (106), un manchon intérieur (107) et un cylindre de lubrification (108), l'embout de scalpel à ultrasons (101) étant situé à l'intérieur du cylindre de lubrification (108).

10. Ensemble de propagation de vibrations ultrasonores (202) selon la revendication 9, dans lequel le cylindre de lubrification (108) est un boîtier en polytétrafluoroéthylène.

11. Système de coupe et d'hémostase à ultrasons, comprenant un ensemble de propagation de vibrations ultrasonores selon l'une des revendications 1 à 10.

12. Système de coupe et d'hémostase à ultrasons selon la revendication 11, comprenant en outre un hôte (201), une poignée (203), un transducteur à ultrasons (204) et un commutateur au pied ou un bouton, dans lequel la poignée (203) comprend un commutateur de serrage (105), l'embout de scalpel à ultrasons (101) de l'ensemble de propagation de vibrations ultrasonores est reliée de manière amovible au transducteur ultrasonore (204) par une partie de connexion (13) à l'extrémité arrière de l'embout de scalpel à ultrasons, et l'hôte (201) est connecté électriquement au transducteur ultrasonore (204) par le biais d'un câble.
